(19) Europäisches Patentamt / European Patent Office / Office européen des brevets

(11) EP 4 574 835 A1

(12) EUROPEAN PATENT APPLICATION
published in accordance with Art. 153(4) EPC

(43) Date of publication:
**25.06.2025 Bulletin 2025/26**

(21) Application number: **23876499.7**

(22) Date of filing: **21.09.2023**

(51) International Patent Classification (IPC):
***C07K 5/087*** (2006.01) ***A61K 38/06*** (2006.01)
***A61P 31/14*** (2006.01)

(52) Cooperative Patent Classification (CPC):
**A61K 38/06; A61P 31/14; C07K 5/0812**

(86) International application number:
**PCT/CN2023/120437**

(87) International publication number:
**WO 2024/078294 (18.04.2024 Gazette 2024/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.10.2022 CN 202211254170**

(71) Applicant: **Guangdong Raynovent Biotech Co., Ltd.**
**Huangpu District**
**Guangzhou 510700 (CN)**

(72) Inventors:
- **CHEN, Xiaoxin**
  **Guangzhou, Guangdong 510700 (CN)**
- **LIU, Chengwu**
  **Guangzhou, Guangdong 510700 (CN)**
- **LIU, Zhuowei**
  **Guangzhou, Guangdong 510700 (CN)**
- **HUANG, Jianzhou**
  **Guangzhou, Guangdong 510700 (CN)**
- **PANG, Daolin**
  **Guangzhou, Guangdong 510700 (CN)**
- **LI, Bo**
  **Guangzhou, Guangdong 510700 (CN)**
- **ZHOU, Guangqiang**
  **Guangzhou, Guangdong 510700 (CN)**
- **CAI, Zemian**
  **Guangzhou, Guangdong 510700 (CN)**
- **LONG, Chaofeng**
  **Guangzhou, Guangdong 510700 (CN)**

(74) Representative: **Potter Clarkson**
**Chapel Quarter**
**Mount Street**
**Nottingham NG1 6HQ (GB)**

# (54) AMORPHOUS FORM OF KETOAMIDE DERIVATIVE, AND PREPARATION METHOD THEREFOR

(57) An amorphous form of a ketoamide derivative and a preparation method therefor. The amorphous form has good stability, thus has a certain medicinal prospect, and provides a feasible active pharmaceutical ingredient for subsequent drug development.

**Description**

## TECHNICAL FIELD

The present application relates to an amorphous form of a ketoamide derivative and a preparation method therefor, as well as an active pharmaceutical ingredient and a pharmaceutical composition comprising the amorphous form.

## BACKGROUND

Before 2002, coronavirus was only regarded as a minor human pathogen, accounting for about 15-25% of common colds. However, the emergence of the severe outbreak of SARS in 2002 caused by the novel coronavirus SARS-CoV has prompted public awareness of diseases caused by coronaviruses. To date, there have been seven known zoonotic coronaviruses that can cause diseases in humans, among which MERS-CoV, SARS-CoV, and SARS-CoV-2 have been identified as the cause of severe acute respiratory syndrome. The pathogen of COVID-19 is SARS-CoV-2 (severe acute respiratory syndrome coronavirus 2), a member of coronaviridae, which can cause respiratory, hepatic, intestinal and neurological diseases in mammals. Symptoms of infection range from asymptomatic diseases to moderate and severe pneumonia, as well as life-threatening complications, including hypoxic respiratory failure, acute respiratory distress syndrome, multiple system organ failure, and ultimately death. What's more, this virus is not only highly contagious, but can be transmitted through asymptomatic infected individuals and those in the symptomatic and pre-symptomatic stages.

3CL protease (3C-like protease, 3CLPro, as known as Mpro) is the main protease responsible for cleaving and processing RNA in the self-encoding of the virus. Most functional (non-structural) proteins of the coronavirus are encoded by the ORF1ab gene, which is initially translated into a polyprotein (7096 amino acids), and then cleaved by the 3CLPro into several active proteins, such as the viral replication protein RdRp. In addition, the protein may cleave the intracellular protein NEMO, thereby inhibiting the activation of the interferon signaling pathway. Therefore, the infection and replication of the virus can be effectively inhibited through the inhibition of 3CLPro. In addition, it has been reported by studies that 3CLPro can also lyse host immune-related proteins, such as human innate immune molecule STING. 3CLPro is not only necessary to maintain the replication of the virus itself, but can also disrupt the immune system of the host cells and inhibit the anti-infection immune responses, thus leading to immune evasion. Inhibition of 3CLPro can not only kill the coronaviruses effectively, but also reduce the immune imbalance within the infected host cells. Given the potent inhibitory or killing effect of 3CLPro inhibitors on coronaviruses and their crucial role in the gene replication process of coronaviruses, 3CLPro inhibitors have become attractive targets in the field of anti-viral chemotherapy.

The following compounds are the 3CLPro inhibitor compounds that have been reported.

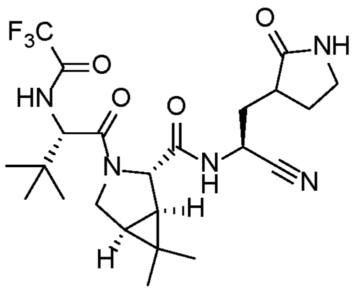

PF-07321332

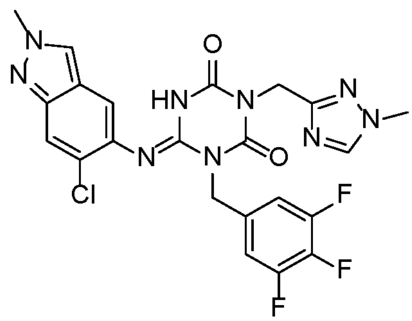

S-217622

A series of ketoamide derivatives have been reported in Patent Application No. PCT/CN2022/117124. *In vitro* activity data show that some of the compounds have good *in vitro* anti-coronaviral activity at the cellular level without cytotoxicity and with significantly higher exposure, slower clearance rate, longer half-life and better pharmacokinetic properties. Among them, compound 1 (Example 1, Formula (I)) shows relatively outstanding overall performance and is considered to have better medicinal prospect.

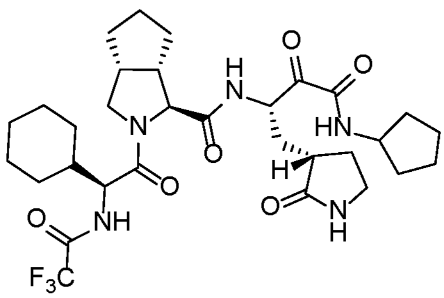

Formula (I)

Crystal form screening is one of the most important aspects of drug development. Given a particular compound, the physicochemical properties of its free form, various salt forms, and corresponding crystal forms are not predictable. Based on further consideration on its druggability, it is of great significance to find suitable crystal forms or amorphous forms to provide multiple intermediate and/or active pharmaceutical ingredient choices for subsequent drug development.

## SUMMARY OF THE INVENTION

In order to overcome the shortcomings of the prior art, a first object of the present application is to provide an amorphous form of a compound of Formula (I). The amorphous form has good stability and good *in vitro* activity against the Mpro protease of novel coronavirus, thus having a certain medicinal prospect and providing a feasible active pharmaceutical ingredient choice for developing the compound of Formula (I) into a clinical medicine.

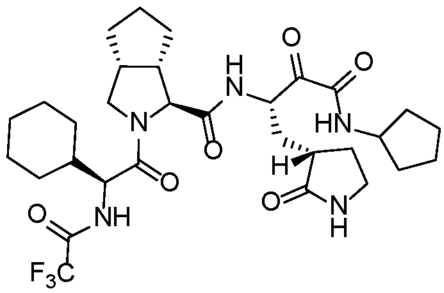

(I)

The above object of the present application is achieved through the following technical solution.

An amorphous form of a compound of Formula (I), wherein, there are no sharp diffraction peaks in an X-ray powder diffraction (XRPD) pattern of the amorphous form.

It is well known to those skilled in the art that, in addition to crystalline state, drugs may exist as an amorphous state, and as a special form of the solid matter, the amorphous form of the compound plays an important role in the drug preparation. Generally, due to the orderly and periodic arrangement of molecules in crystalline matters, the energy of intermolecular interaction is reduced, resulting in low energy. In contrast, molecules in the amorphous state are in a highly disordered state, resulting in greater surface free energy of the substance. The molecules in amorphous solids have higher energy compared to those in crystalline solids, making them more likely to disperse, thus increasing their dissolution rate and improving the bioavailability of the drug. Amorphous form is considered to be at thermodynamically high-energy state and have a thermodynamically metastable structure. The fundamental particles constituting amorphous compounds are arranged in a disordered manner in three-dimensional space, and X-ray powder diffraction pattern is one of the most intuitive ways for identifying the amorphous forms. Particularly, when a compound exists in an amorphous form, there are generally no sharp diffraction peaks in its X-ray powder diffraction (XRPD) pattern, that is, there are no diffraction peaks, or there is one or several broad diffraction peaks in the XRPD pattern. It is understood by those skilled in the art that the broad diffraction peaks in the XRPD pattern of the amorphous form are identified relative to the narrow and sharp diffraction peaks in the XRPD pattern of the crystal form. Generally, the broad diffraction peaks in the XRPD pattern of the amorphous form span a 2$\theta$ angle of up to 5° or even larger.

Particularly, there are two broad diffraction peaks respectively at 2$\theta$ angles between 5°-15° and between 15°-25° in the X-ray powder diffraction pattern of the amorphous form of the compound of Formula (I).

In a specific embodiment of the present application, the X-ray powder diffraction pattern of the amorphous form of

the compound of Formula (I) is essentially shown in Figure 1.

In some embodiments of the present application, there is a starting point of an endothermic peak at 71.35±3 °C on a differential scanning calorimetry (DSC) curve of the amorphous form.

In a specific embodiment of the present application, the DSC pattern of the amorphous form is shown in Figure 2.

In some embodiments of the present application, there is a weight loss of up to 2.209% at 100.00±3 °C on a thermogravimetric analysis (TGA) curve of the amorphous form.

In a specific embodiment of the present application, the TGA pattern of the amorphous form is shown in Figure 3.

In another specific embodiment of the present application, the X-ray powder diffraction pattern of the amorphous form of the compound of Formula (I) is shown in Figure 4.

In another specific embodiment of the present application, the X-ray powder diffraction pattern of the amorphous form of the compound of Formula (I) is shown in Figure 5.

A second object of the present application is to provide a method for preparing an amorphous form of a compound of Formula (I), which comprises: suspending and slurrying the compound of Formula (I) in a solvent to obtain a slurry system, centrifuging the slurry system to precipitate a solid, and then drying the solid under vacuum at room temperature; wherein the solvent is selected from a group consisting of n-hexane, n-heptane, cyclohexane, water, petroleum ether, and ethyl formate, the slurrying is performed at a temperature of 25-45 °C for a period of 48-72 h, and a mass/volume ratio of the compound to the solvent in the preparation method is 1 g: 0.5-2.5 mL.

This method has the advantages of stable process, wild reaction conditions and readily available raw materials, and thus can be used for mass industrial production of the amorphous form of the compound of Formula (I).

The present application further provides an active pharmaceutical ingredient comprising the amorphous form of the compound of Formula (I) of the present application.

The present application further provides a pharmaceutical composition comprising the amorphous form of the compound of Formula (I) and at least one pharmaceutically acceptable carrier or excipient. The pharmaceutical composition is formulated as an oral preparation or an injection.

Preferably, the oral preparation is in a form of powder, granule, pellet, capsule, tablet, solution or troche. The pharmaceutical composition is composed of the active pharmaceutical ingredient and a pharmaceutically acceptable excipient. The pharmaceutically acceptable carrier or excipient comprises, but not limited to, at least one of filler, binder, disintegrant, lubricant and the like.

Particularly, based on the beneficial effects of the amorphous form of the compound of Formula (I) in the present application, the beneficial effects are ultimately reflected in the pharmaceutical composition. More particularly, the pharmaceutical composition comprises a mass percentage of any value from 1.00% to 99.00% of the active pharmaceutical ingredient, further, the pharmaceutical composition comprises a mass percentage of any value from 5.00% to 95.00% of the active pharmaceutical ingredient, and more further, the pharmaceutical composition comprises a mass percentage of any value from 10.00% to 90.00% of the active pharmaceutical ingredient.

The present application further provides use of a pharmaceutical composition containing the amorphous form of the compound of Formula (I) in the preparation of a product for preventing, alleviating or treating an infection or a disease caused by a novel coronavirus. The novel coronavirus comprises non-variants or variants of the novel coronavirus.

Preferably, the variants of the novel coronavirus comprise novel coronavirus Alpha variant, novel coronavirus Beta variant, novel coronavirus Gamma variant, novel coronavirus Delta variant, novel coronavirus Lambda variant and/or novel coronavirus Omicron variant. The infection comprises fever, cough, sore throat, pneumonia, acute respiratory infection, severe acute respiratory infection, hypoxic respiratory failure, acute respiratory distress syndrome, sepsis or septic shock. Preferably, the disease comprises novel coronavirus pneumonia.

In summary, the amorphous form of the compound of Formula (I) of the present application has a certain medicinal prospect. Therefore, if it is demonstrated by detection means that the amorphous form of the compound of Formula (I) exists in the active pharmaceutical ingredient and/or in the pharmaceutical composition, it shall be deemed that the amorphous form of the compound of Formula (I) as provided in the present application has been used. In addition to X-ray powder diffraction as mentioned above, the detection means may further include such methods as Differential Scanning Calorimetry (DSC), Infrared Spectroscopy (IR), Raman Spectrometry (Raman), Solid State Nuclear Magnetic Resonance (SSNMR), as well as all other detection methods that, individually or in combination, can be used to demonstrate the use of the amorphous form of the compound of Formula (I) of the present application, and the effects resulted from pharmaceutical excipients can be removed by using methods commonly used by those skilled in the art, such as subtractive pattern.

With respect to the prior art, the present application has the following advantages and beneficial effects:

1. Firstly disclosing an amorphous form of a compound of Formula (I) and a preparation method therefor. Unlike the compounds that conventionally exist in an amorphous form with poor stability, poor medicinal properties and other defects, the amorphous form of the compound of Formula (I) of the present application exhibits high stability, which is specifically demonstrated by the high stability of the compound of formula (I) under conditions of high temperature and

high humidity, as well as good *in vitro* activity against the Mpro protease of novel coronavirus. Therefore, it can be determined based on the disclosed stability data that the amorphous form of the compound of Formula (I) has a certain medicinal prospect.

2. Providing an active pharmaceutical ingredient comprising the amorphous form of the compound of Formula (I) of the present application.

3. Providing a pharmaceutical composition comprising the amorphous form of the compound of Formula (I) and at least one pharmaceutically acceptable carrier.

4. Providing use of the pharmaceutical composition comprising the amorphous form of the compound of Formula (I) in the preparation of a product for preventing, alleviating or treating an infection or a disease caused by a novel coronavirus. The amorphous form of the compound of Formula (I) involved in the present application has significant inhibition on the Mpro protease of novel coronavirus *in vitro,* and it is found by a pharmacokinetic model of mouse that the compound of Formula (I) shows a lower clearance rate, a longer half-life, and better pharmacokinetic properties.

5. Providing an amorphous form of a compound of Formula (I) and a preparation method therefor, thus providing multiple intermediate and/or active pharmaceutical ingredient choices for the scale production of active pharmaceutical ingredients and the downstream process (e.g., formulation process) of pharmaceutical products.

6. Providing a pharmaceutical composition composed of an active pharmaceutical ingredient of the present application and a pharmaceutically acceptable excipient, which has consistent beneficial effects with the amorphous form of the compound of Formula (I) of the present application.

## Definition and illustration

Unless otherwise indicated, the following terms and phrases used herein are intended to have the meanings below. A particular phrase or term should not be considered indefinite or unclear in the absence of a particular definition, but should be understood according to its common meaning. When a trade name is mentioned herein, it is intended to refer to its corresponding commodity or its active ingredient.

The intermediate compounds of the present application can be prepared by a variety of synthetic methods well known to those skilled in the art, including the specific embodiments listed below, the embodiments formed by combining the specific embodiments with other chemical synthetic methods, as well as equivalent replacements well known to those skilled in the art, and the preferred embodiments include but are not limited to the embodiments disclosed in the present application.

The chemical reactions in the specific embodiments of the present application are carried out in suitable solvents, which must be suitable for the chemical changes of the present application and the reagents and materials required therefor. In order to obtain the compounds of the present application, it is sometimes necessary for the person skilled in the art to modify or select the synthesis steps or the reaction process on the basis of the disclosed embodiments.

The present application will be described in detail below by means of embodiments, which do not imply any limitation of the present application.

All solvents used in the present application are commercially available and can be used directly without further purification.

The solvents used in the present application are commercially available. The following abbreviations are used herein: NaCl represents sodium chloride; DCM represents dichloromethane; DMF represents N,N-dimethylformamide; DMSO represents dimethylsulfoxide; MeOH represents methanol; TFA represents trifluoroacetic acid; EDTA represents ethylenediamine tetraacetic acid; mp represents melting point.

1.1 X-ray powder diffraction (X-ray powder diffractometer, XRPD)

Instrument model: Bruker D8 Advance X-ray powder diffractometer
Test method: about 10-20 mg of sample is used for XRPD detection.

The detailed XRPD parameters are as follows:

Tube: Cu, k$\alpha$, ($\lambda$=1.54056Å)
Tube voltage: 40 kV, Tube current: 40 mA
Divergence slit: 0.60 mm
Detector slit: 10.50 mm
Anti-scattering slit: 7.10 mm
Scanning range: 4-40 deg

Step angle: 0.02 deg
Step size: 0.12 sec
Rotation speed of sample plate: 15 rpm

1.2 Differential thermal analysis (Differential Scanning Calorimeter, DSC)

Instrument model: TA Q2000 Differential Scanning Calorimeter
Test method: sample (approximately 1 mg) is tested in an aluminum pot for DSC, for which the method is to heat the sample from 25 °C to 350 °C at a heating rate of 10 °C/min under a condition of 50 mL/min $N_2$.

1.3 Thermogravimetric analysis (Thermal Gravimetric Analyzer, TGA)

Instrument model: TA Q5000IR Thermal Gravimetric Analyzer
Test method: A sample (2-5mg) is tested in a platinum pot for TGA, for which the method is to heat the sample from room temperature to 350 °C at a heating rate of 10 °C/min under a condition of 25mL/min $N_2$.

**BRIEF DESCRIPTION OF THE DRAWINGS**

Figure 1 shows an XRPD pattern under Cu-Kα radiation of the amorphous form of the compound of Formula (I) in Example 2.
Figure 2 shows a DSC pattern of the amorphous form of the compound of Formula (I).
Figure 3 shows a TGA pattern of the amorphous form of the compound of Formula (I).
Figure 4 shows an XRPD pattern under Cu-Kα radiation of the amorphous form of the compound of Formula (I) in Example 3.
Figure 5 shows an XRPD pattern under Cu-Kα radiation of the amorphous form of the compound of Formula (I) in Example 4.

**DETAILED DESCRIPTION**

The present application will be described in detail below by means of embodiments, which do not imply any adverse limitation to the present application. The present application has been described in detail herein, wherein specific embodiments thereof are also disclosed, and it will be apparent to those skilled in the art that various changes and modifications may be made with respect to specific embodiments of the present application without departing from the essence and scope of the present application.

Example 1: Preparation of compound of Formula (I)

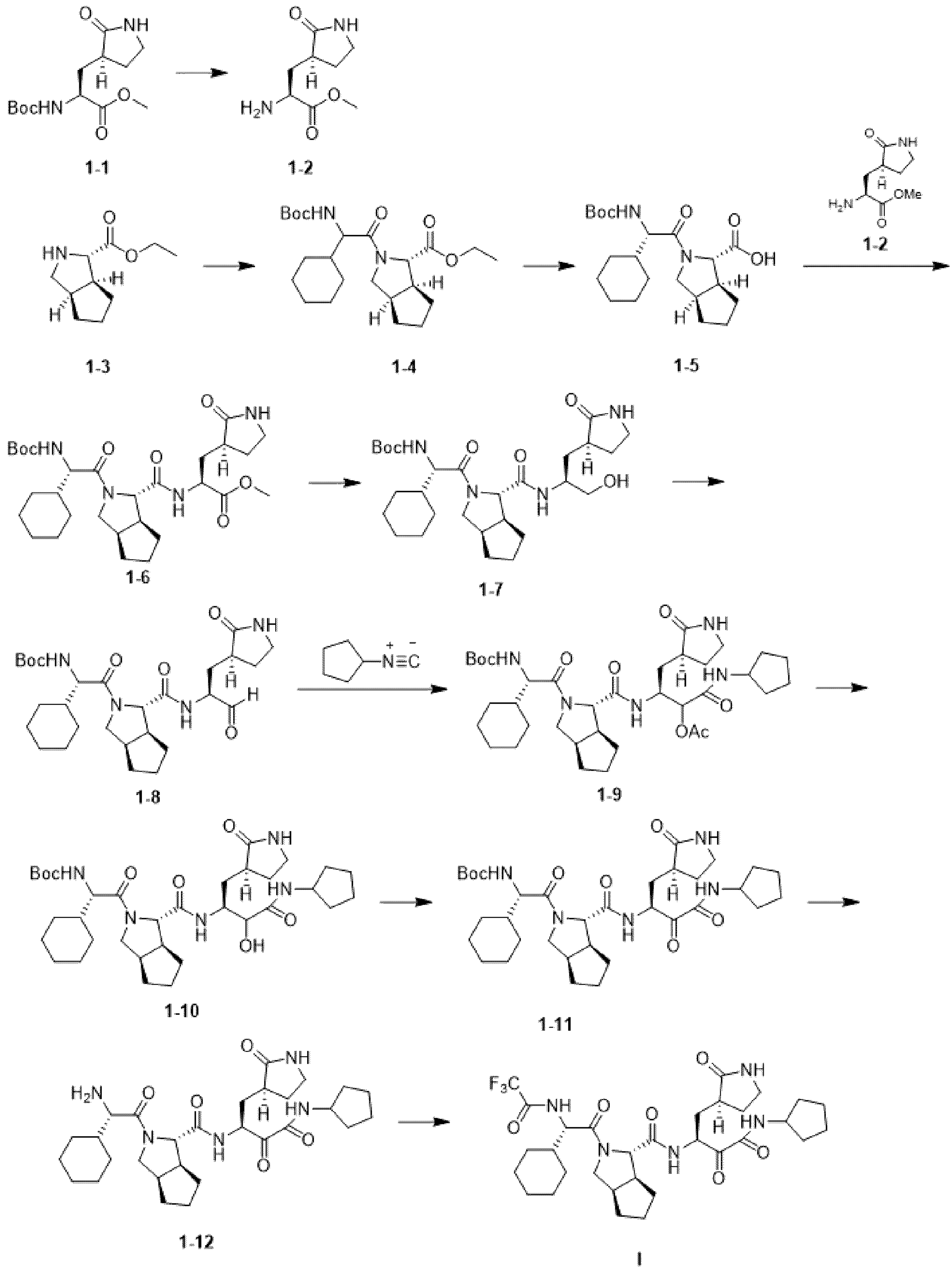

1-1 1-2 1-3 1-4 1-5 1-2 1-6 1-7 1-8 1-9 1-10 1-11 1-12 I

Step 1: Synthesis of hydrochloride of Compound 1-2

Compound 1-1 (500 mg, 1.75 mmol) was dissolved in ethyl acetate (5 mL), into which was added a solution of hydrogen chloride in ethyl acetate (10 mL, 4 N) to react while stirring at 20 °C for 2 h. The reaction solution was

concentrated under reduced pressure to obtain a hydrochloride of Compound 1-2, without purification. $^1$H NMR (400 MHz, $CD_3OD$) δ = 4.28 - 4.20 (m, 1H), 3.91 - 3.81 (m, 3H), 3.45 - 3.35 (m, 2H), 2.86 - 2.74 (m, 1H), 2.48 - 2.36 (m, 1H), 2.29 - 2.19 (m, 1H), 2.02 - 1.94 (m, 1H), 1.93 - 1.80 (m, 1H).

Step 2: Synthesis of Compound 1-4

A compound Boc-L-cyclohexyl glycine (1 g, 3.89 mmol) was added into N,N-dimethylformamide (10 mL), into which was added 2-(7-azabenzotriazol-1-yl)-*N,N,N,N*-tetramethyluronium hexafluorophosphate (1.77 g, 4.66 mmol) to react while stirring for 0.5 h, and then added diisopropylethylamine (1.26 g, 9.72 mmol) and the hydrochloride of Compound 1-3 (1.02 g, 4.66 mmol) to react while stirring at 20 °C for 16 h. Into the reaction solution was added methyl tert-butyl ether (50 mL), and then the reaction solution was washed with water (20 mL), 3% citric acid (20 mL ×2), and saturated sodium chloride solution (20mL). The organic layer was dried over anhydrous sodium sulfate, filtered, concentrated under reduced pressure, and purified by silica gel column chromatography (petroleum ether: ethyl acetate = 3:1) to obtain Compound 1-4. $^1$H NMR (400MHz, $CDCl_3$) δ = 5.22 - 5.11 (m, 1H), 4.36 (d, *J*=3.9 Hz, 1H), 4.27 (dd, *J*=6.9, 9.3 Hz, 1H), 4.21 - 4.12 (m, 2H), 3.83 (dd, *J*=7.8, 10.4 Hz, 1H), 3.70 (br dd, *J*=3.6, 10.4 Hz, 1H), 2.81 - 2.61 (m, 2H), 1.82 - 1.70 (m, 6H), 1.68 - 1.61 (m, 4H), 1.56 - 1.48 (m, 2H), 1.46 - 1.38 (m, 9H), 1.29 - 1.22 (m, 4H), 1.21 - 0.98 (m, 4H).

Step 3: Synthesis of Compound 1-5

Compound 1-4 (1.41 g, 3.34 mmol) was added into tetrahydrofuran (14 mL), into which was added a solution of lithium hydroxide monohydrate $LiOH \cdot H_2O$ (280.03 mg, 6.67 mmol) in water (5 mL) to react while stirring at 20 °C for 16 h. The crude product was neutralized with 3% citric acid solution (50 mL), and extracted with ethyl acetate (50 mL). The organic phase was washed with saturated sodium chloride solution (30 mL). The organic layer was dried over anhydrous sodium sulfate, filtered, and concentrated under reduced pressure to obtain Compound 1-5, without purification. $^1$H NMR (400MHz, DMSO-$d_6$) δ = 12.58 - 12.23 (m, 1H), 6.92 - 6.82 (m, 1H), 4.11 - 3.94 (m, 2H), 3.82 - 3.76 (m, 1H), 3.72 - 3.62 (m, 1H), 2.73 - 2.64 (m, 1H), 2.62 - 2.55 (m, 1H), 1.92 - 1.42 (m, 12H), 1.40 - 1.32 (m, 9H), 1.18 - 1.06 (m, 3H), 1.00 - 0.81 (m, 2H).

Step 4: Synthesis of Compound 1-6

Compound 1-5 (650 mg, 1.65 mmol) was added into 2-butanone (7mL), into which were added 1-hydroxybenzotriazole (222.63 mg, 1.65 mmol), 1-(3-dimethylaminopropyl)-3-ethylcarbodiimidehydrochloride (379.03 mg, 1.98 mmol), and diisopropylethylamine (638.84 mg, 4.94 mmol) to react while stirring at 20 °C for 0.5 h, and then added the hydrochloride of Compound 1-2 (366.88 mg, 1.65 mmol) to react while stirring at 20 °C for 16 h. Into the reaction solution was added water (20 mL), and then the reaction solution was extracted with dichloromethane: methanol (30 mL×2, 10:1). The organic phase was combined, and washed with 3% citric acid (20 mL×2) and saturated sodium chloride solution (20 mL). The organic layer was dried over anhydrous sodium sulfate, filtered, concentrated under reduced pressure, and purified by silica gel column chromatography (dichloromethane: methanol= 20: 1) to obtain Compound 1-6. $^1$H NMR (400 MHz, $CDCl_3$) δ = 7.49 - 7.42 (m, 1H), 6.23 - 6.05 (m, 1H), 5.28 - 5.17 (m, 1H), 4.64 - 4.51 (m, 1H), 4.43 - 4.24 (m, 2H), 3.92 - 3.81 (m, 1H), 3.78 - 3.70 (m, 3H), 3.39 - 3.27 (m, 2H), 2.94 - 2.75 (m, 2H), 2.57 - 2.36 (m, 2H), 2.24 - 2.07 (m, 1H), 1.94 - 1.50 (m, 14H), 1.49 - 1.41 (m, 9H), 1.27 - 0.95 (m, 6H).

Step 5: Synthesis of Compound 1-7

Compound 1-6 (3.10 g, 5.51 mmol) was dissolved in tetrahydrofuran (31 mL), into which was added lithium borohydride (240.02 mg, 11.02 mmol) at 0 °C and warmed to 20 °C slowly to react for 2 h. Into the reaction solution were added water (10 mL) and ethyl acetate (20mL), and the reaction solution was stirred for 10 min. White solid was precipitated and filtered to obtain a filter cake, which was a crude product of Compound 1-7. [M+1]+ = 535.4.

Step 6: Synthesis of Compound 1-8

Compound 1-7 (0.5 g, 935.13 μmol) was dissolved in dichloromethane (10 mL), and Dess-Martin periodinane (594.94mg, 1.40mmol) was then added into the reaction system to react while stirring at 25 °C for 16 h. Saturated sodium thiosulfate (15 mL) and saturated sodium bicarbonate solution (15 mL) were added into the reaction system, which was then stirred for 10 min and extracted with dichloromethane (50 mL ×2). The organic phase was washed with saturated salt water (15 mL), dried over anhydrous sodium sulfate, filtered, and concentrated to obtain a crude product of Compound 1-8. [M+1]+ = 533.4.

Step 7: Synthesis of Compound 1-9

Compound 1-8 (436 mg, 818.52 μmol) was dissolved in dichloromethane (5 mL). Glacial acetic acid (58.98 mg, 982.22 mmol) and cyclopentyl isocyanate (94.44 mg, 982.22 μmol) were added into the reaction system to react while stirring at 25 °C for 2 h. Saturated ammonium chloride solution (10 mL) was added into the reaction system, which was then stirred for 10 min and extracted with dichloromethane (20 mL). The organic phase was washed with water (10 mL), dried over anhydrous sodium sulfate, filtered, concentrated under reduced pressure, and purified by silica gel column chromatography (dichloromethane: methanol =10: 1) to obtain Compound 1-9. [M+1]+ = 688.4.

Step 8: Synthesis of Compound 1-10

Compound 1-9 (190 mg, 276.22 μmol) was dissolved in methanol (3 mL), into which was then added a solution of potassium carbonate (95.44 mg, 690.54 μmol) in water (2 mL) to react while stirring at 20 °C for 16 h. 3% citric acid (20 mL) was added into the reaction system, which was then extracted with dichloromethane (40 mL) three times. The organic phase was washed with saturated sodium chloride solution (30 mL), dried over anhydrous sodium sulfate, filtered, and concentrated under reduced pressure to obtain a crude product of Compound 1-10. $[M+1]^+$ = 646.5.

Step 9: Synthesis of Compound 1-11

Compound 1-10 (238.00 mg, 368.52 μmol) was dissolved in dichloromethane (24 mL), into which was then added Dess-Martin periodinane (203.19 mg, 479.08 μmol) to react while stirring at 20 °C for 18 h. Sodium thiosulfate (15 mL) and sodium bicarbonate solution (15 mL) were added into the reaction system, which was then stirred for 10 min and extracted with dichloromethane (50 mL ×2). The organic phase was washed with saturated salt water (15 mL), dried over anhydrous sodium sulfate, filtered, concentrated, and purified by silica gel column chromatography (dichloromethane: methanol= 20: 1) to obtain the product of Compound 1-11. $[M+1]^+$ = 644.5.

Step 10: Synthesis of Compound 1-12

Compound 1-11 (125 mg, 194.16 μmol) was dissolved in tetrahydrofuran (3 mL), into which was then added ethyl acetate hydrochloride (4 mol/L, 2.91 mL) to react while stirring at 20 °C for 1 h. The reaction solution was directly rotary evaporated with an oil pump, and then rotary evaporated again with a small amount of dichloromethane to obtain Compound 1-12. $[M+1]^+$ =544.4.

Step 11: Synthesis of Compound I

Compound 1-12 (125 mg, 229.91 μmol) was dissolved in tetrahydrofuran (2.5 mL) at 0 °C, into which were added trifluoroacetic anhydride (193.15 mg, 919.63 μmol) and pyridine (127.30 mg, 1.61 mmol) to react while stirring at 20 °C for 16 h. Into the reaction system was added water (20mL), and the reaction system was extracted with dichloromethane (40 mL ×2). The organic phase was washed with 3% citric acid (40 mL) and saturated sodium chloride solution (40 mL ×2) successively, dried over anhydrous sodium sulfate, filtered, and concentrated. The crude product was separated by preparative HPLC to obtain Compound I. $[M+1]^+$ = 640.0, $^1H$ NMR (400 MHz, $CD_3OD$) δ ppm 0.94 - 1.10 (m, 2 H), 1.13 - 1.32 (m, 3 H) 1.32 - 1.46 (m, 1 H), 1.47 - 1.57 (m, 3 H), 1.59 - 1.68 (m, 4 H), 1.69 - 1.81 (m, 6 H), 1.83 - 2.00 (m, 5 H), 2.01 - 2.17 (m, 1 H), 2.19 - 2.38 (m, 1 H), 2.49 - 2.57 (m, 1 H), 2.58 - 2.70 (m, 1 H), 2.73 - 2.89 (m, 1 H), 3.20 - 3.26 (m, 1 H), 3.37 - 3.45 (m, 1 H), 3.73 - 3.86 (m, 1 H), 3.88 - 3.97 (m, 1 H), 4.03 - 4.10 (m, 1 H), 4.11 - 4.18 (m, 1 H), 4.19 - 4.29 (m, 1 H), 4.29 - 4.37 (m, 1 H), 4.39 - 4.47 (m, 1 H), 4.57 - 4.60 (m, 2 H).

Example 2: Preparation of amorphous form of the compound of Formula (I)

20.3 mg of the compound of Formula (I) was weighed, into which was dropwise added 10.0 mL of n-heptane at room temperature, and suspended and slurried at 25 °C for 72 h. A resulting slurry system was centrifuged to precipitate a solid, and the solid precipitation was dried under vacuum at room temperature, and detected by XRPD for its morphology, which showed that the morphology of the resulting final product was amorphous.

For the resulting final product, the XRPD pattern under Cu-Kα radiation was shown in Figure 1; the DSC pattern was shown in Figure 2; and the TGA pattern was shown in Figure 3.

Example 3: Preparation of amorphous form of the compound of Formula (I)

19.8 mg of the compound of Formula (I) was weighed, into which was dropwise added 10.0 mL of n-hexane at

room temperature, and suspended and slurried at 25 °C for 72 h. A resulting slurry system was centrifuged to precipitate a solid, and the solid precipitation was dried under vacuum at room temperature, and detected by XRPD for its morphology, which showed that the morphology of the resulting final product was amorphous. The resulting final product was the same amorphous form as that in Example 2. The XRPD pattern under Cu-Kα radiation of the resulting final product was shown in Figure 4.

Example 4: Preparation of amorphous form of the compound of Formula (I)

20.3 mg of the compound of Formula (I) was weighed, into which was dropwise added 10.0 mL of water at room temperature, and suspended and slurried at 25 °C for 72 h. A resulting slurry system was centrifuged to precipitate a solid, and the solid precipitation was dried under vacuum at room temperature, and detected by XRPD for its morphology, which showed that the morphology of the resulting final product was amorphous. The resulting final product was the same amorphous form as that in Example 2. The XRPD pattern under Cu-Kα radiation of the resulting final product was shown in Figure 5.

Example 5: Test on the solid stability of the amorphous form of the compound of Formula (I) under conditions of high temperature and high humidity

2 samples of the amorphous form of the compound of Formula (I) were weighed in parallel, approximately 100 mg each, and placed in the bottom of a glass sample vial and spread into a thin layer. The sample vial was sealed with aluminum foil having small holes poked therein to ensure that the samples can be in full contact with the ambient air, and placed in a box with constant temperature and humidity at conditions of 40°C/75% humidity. The samples placed under the above conditions were tested on Days 0, 12, and 30, and the test results were compared with the initial test result on Day 0 and shown in Table 1 below:

Table 1. Test on the solid stability of the amorphous form of the compound of Formula (I)

| Holding conditions | | | Day 0 | High temperature (Day) | | High humidity (Day) | | Room temperature (Day) | | High temperature and high humidity (Day) | |
|---|---|---|---|---|---|---|---|---|---|---|---|
| Holding time | | | | 12 | 30 | 12 | 30 | 12 | 30 | 12 | 30 |
| Characteristics | Appearance | Should be white to yellowish powder | White powder | White powder | White powder | White powder | White powder | White powder | White powder | White powder | White powder |
| Detection | Total impurities | ≤5.0% | 1.0 | 1.8 | 2.9 | 1.3 | 1.8 | 1.1 | 1.0 | 1.5 | 1.2 |
| | Water | ≤5.0% | 3.80 | 1.01 | 1.63 | 6.65 | 6.96 | 3.92 | 4.45 | 3.96 | 4.43 |
| Content | | 95.0%-102.0% | 100.0 | 97.1 | 94.3 | 98.7 | 96.8 | 99.9 | 98.8 | 98.8 | 97.5 |
| Crystal form | | Should be consistent with the control | Consistent with the control | Consistent with the control | Consistent with the control | Consistent with the control | Consistent with the control | Consistent with the control | Consistent with the control | Consistent with the control | Consistent with the control |
| Weight gain after moisture absorption | | Reported value % | N/A | N/A | N/A | 5.0 | 5.0 | N/A | N/A | N/A | N/A |

The detection items of the product were compared with those on Day 0.

After holding at a high temperature of 60 °C for 30 days, there was no difference in the crystal form, no difference in the characteristics, a slight increase in the total impurities, an obvious decrease in the water content, and a slight decrease in the content of the product.

After holding at a high humidity of 92.5% RH for 30 days, there was no difference in the crystal form, no difference in the characteristics, a slight increase in the total impurities, an obvious increase in the water content, and a slight decrease in the content of the product. After holding in an open environment for 30 days under a high humidity condition, the samples showed a weight gain of 5.0% due to moisture absorption, indicating the hygroscopicity in nature.

After holding under conditions of room temperature 25 °C and 60% RH for 30 days, there was a slight increase in the water content, and no obvious difference in the remaining detection data.

After holding at a high temperature of 40 °C and a high humidity of 75% RH for 30 days, there was no difference in the crystal form, no difference in the characteristics, no obvious difference in the total impurities, a slight increase in the water content, and a slight decrease in the content of the product.

Compared with the compound of Formula (I) prepared in Example 1, the amorphous form of the compound of Formula (I) is relatively stable under conditions of high temperature, high humidity, and a combination of high temperature and high humidity, and even more stable at room temperature.

In conclusion, when holding in an open environment, the amorphous form of the compound of Formula (I) is relatively stable under conditions of high temperature, high humidity, and both high temperature and high humidity, and even more stable at room temperature.

Example 6: Test on the stability of the amorphous form of the compound of Formula (I) in different solvents

Multiple samples of the amorphous form of the compound of Formula (I) were taken, approximately 20 mg each, into which were respectively added 1.0-2.5 mL of the solvents listed in the table below, and the mixture was stirred at 40 °C. After stirring for 2 days, if the sample was in solution or close to a solution state, the solvent was removed by filtration and natural evaporation. If the sample was still in suspension, the sample was centrifuged, the precipitate was collected, and the supernatant was placed in a fume hood to evaporate until the solvent evaporates to dry. The obtained precipitate and the solid left behind after drying the solvent were dried in a vacuum drying oven at 40 °C overnight. All the solid in the sample was collected, for which the characteristic was detected by XRPD. The results are shown in Table 2.

Table 2. Test on the stability of the amorphous form of the compound of Formula (I) in different solvents

| No. | Solvents | Appearance | Result |
|---|---|---|---|
| 1 | Toluene | Suspension (Day 3)/solid precipitates after removing the solvent by evaporation | All amorphous form |
| 2 | Dichloromethane | Suspension (Day 3)/solid precipitates after removing the solvent by evaporation | All amorphous form |
| 3 | Methanol | Suspension (Day 3)/solid precipitates after removing the solvent by evaporation | All amorphous form |
| 4 | n-hexane | Suspension (Day 3)/solid precipitates after removing the solvent by evaporation | All amorphous form |
| 5 | n-heptane | Suspension (Day 3)/solid precipitates after removing the solvent by evaporation | All amorphous form |

The experimental data demonstrated that, there was no morphologic change of the amorphous form of the compound of Formula (I) occurring in conventional solvents, indicating that the amorphous form has higher stability, making it possible to provide multiple intermediate and/or active pharmaceutical ingredient choices for the scale production of active pharmaceutical ingredients and the downstream process (e.g., formulation process) of pharmaceutical products.

Example 7: *In vitro* test

1. Experimental materials:

1.1 Reagents and consumables, and sources:

Tris: Sigma;
EDTA: Sigma;
NaCl: Sigma;
384-well Plate: Perkin Elmer;
Dimethyl sulfoxide (DMSO): Sigma;
Substrate (Dabcyl-KTSAVLQSGFRKM-(Edans)): GenScript;
SARS-CoV-2 Mpro: WuXi App Tec;
GC376: Target Mol.

1.2 Instrument and sources:

SpectraMax M2e microplate reader: Molecular Devices;
Echo 655 Liquid handling workstation: Labcyte;
Table-top high-speed centrifuge: Eppendorf.

2. Experimental method:

The compound of Formula (I) was dissolved in DMSO, diluted with Echo655 in a 3-fold gradient according to the required concentrations to be tested, generating 10 concentration points with two replicates for each concentration, and then added to a 384-well plate. Mpro protein and the substrate were diluted with test buffer (100 mM NaCl, 20 mM Tris-HCl, 1 mM EDTA). The Mpro protein was then added to the 384-well plate, and incubated with the compound at room temperature for 30 min, into which was then added the substrate, with a test concentration of 25 nmol/L for the Mpro protein and a test concentration of 25 μmol/L for the substrate. The mixture was incubated at 30 °C in a thermostatic incubator for 60 min, and the fluorescence signal value at Ex/Em = 340nm/490nm was detected with a microplate reader. At the same time, the background wells containing the substrate and the compound but without the Mpro protein was also detected as the control.

3. Data analysis:

1) Inhibition rate was calculated using the formula below:

$$\text{Inhibition rate }\%=[(\text{Compound-BG}_{\text{compound}})-(\text{ZPE-BG}_{\text{ZPE}})]/[(\text{HPE-BG}_{\text{HPE}})-(\text{ZPE-BG}_{\text{ZPE}})]\times 100\%$$

\# HPE: 100% inhibition control, comprising 25 nmol/L Mpro protein + 25 μmol/L substrate + 1 μmol/L GC376
ZPE: Without inhibition control, comprising 25 nmol/L Mpro protein + 25 μmol/L substrate, without the compound
Compound: Test compound well, comprising 25 nmol/L Mpro protein + 25 μmol/L substrate + the compound
BG: Background control well, comprising 25 μmol/L substrate + the compound, without the Mpro protein

2) The inhibition rate data (inhibition rate %) of the compounds was analyzed by log(agonist) vs. response - Variable slope nonlinear fitting using GraphPad Prism software, to obtain the $IC_{50}$ values of the compounds, with the experimental results shown in Table 3.

Table 3. *In vitro* activity of test compounds against the Mpro protease of novel coronavirus

| Compound No. | $IC_{50}$ (nM) |
|---|---|
| Compound of Formula (I) | 25 |

Conclusion: The compound of the present application has good *in vitro* activity against the Mpro protease of novel coronavirus.

Based on the above experiments for the evaluation of the stability and activity of the amorphous form of the compound of formula (I), it can be seen that, different from the general perception of the amorphous drugs in the art, the amorphous form of the compound of Formula (I) provided in the present application shows higher stability of chemical properties and physical forms, and the amorphous form of the compound of Formula (I) has a significant inhibitory effect on the Mpro protease of novel coronavirus, indicating that the amorphous form of the compound of Formula (I) has a good medicinal prospect.

The above examples are preferred embodiments of the present application, but the embodiments of the present application are not limited by the above examples, and any other changes, modifications, substitutions, combinations, and simplifications made without departing from the essence and principle of the present application shall be equivalent replacements, and are included in the scope of protection of the present application.

## Claims

1. An amorphous form of a compound of Formula (I),

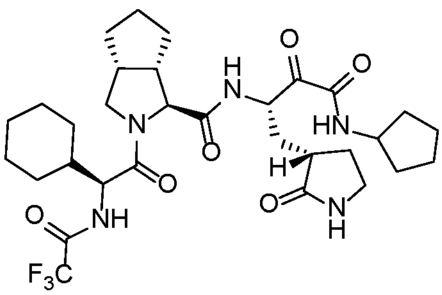

(I)

wherein, there are no sharp diffraction peaks in an X-ray powder diffraction pattern of the amorphous form.

2. The amorphous form of the compound of Formula (I) according to claim 1, wherein, there are two characteristic peaks respectively at 2θ angles between 5°-15° and between 15°-25° in the X-ray powder diffraction pattern of the amorphous form.

3. The amorphous form of the compound of Formula (I) according to claim 1, wherein, the X-ray powder diffraction pattern of the amorphous form is essentially shown in Figure 1.

4. The amorphous form of the compound of Formula (I) according to claim 1, wherein, there is a starting point of an endothermic peak at 71.35±3 °C on a differential scanning calorimetry (DSC) curve of the amorphous form.

5. The amorphous form of the compound of Formula (I) according to claim 4, wherein, the DSC diagram of the amorphous form is shown in Figure 2.

6. The amorphous form of the compound of Formula (I) according to claim 1, wherein, there is a weight loss of up to 2.209% at 100.00±3 °C on a thermogravimetric analysis (TGA) curve of the amorphous form.

7. The amorphous form of the compound of Formula (I) according to claim 6, wherein, the TGA pattern of the amorphous form is shown in Figure 3.

8. A method for preparing an amorphous form of a compound of Formula (I), comprising: suspending and slurrying the compound of Formula (I) in a solvent to obtain a slurry system, centrifuging the slurry system to precipitate a solid, and then drying the solid under vacuum at room temperature;
wherein the solvent is selected from a group consisting of n-heptane, cyclohexane, water, petroleum ether, and ethyl formate, the slurrying is performed at a temperature of 25-45 °C for a period of 48-72 h, and a mass/volume ratio of the compound to the solvent is 1 g: 0.5-2.5 mL.

**9.** A pharmaceutical composition, comprising the amorphous form of the compound of Formula (I) according to any one of claims 1-7 and at least one pharmaceutically acceptable carri er.

**10.** The pharmaceutical composition according to claim 9, wherein the pharmaceutical composition is formulated as an oral preparation or an injection; and preferably, the oral preparation is in a form of powder, granule, pellet, capsule, tablet, solution or troche.

**11.** Use of the pharmaceutical composition of claim 9 or 10 in the preparation of a product for preventing, alleviating or treating an infection or a disease caused by a novel coronavirus.

**12.** The use according to claim 11, wherein the novel coronavirus comprises non-variants or variants of the novel coronavirus.

**13.** The use according to claim 12, wherein the variants of the novel coronavirus comprise novel coronavirus Alpha variant, novel coronavirus Beta variant, novel coronavirus Gamma variant, novel coronavirus Delta variant, novel coronavirus Lambda variant and/or novel coronavirus Omicron variant.

**14.** The use according to any one of claims 11-13, wherein the infection comprises fever, cough, sore throat, pneumonia, acute respiratory infection, severe acute respiratory infection, hypoxic respiratory failure, acute respiratory distress syndrome, sepsis or septic shock; and preferably, the disease comprises novel coronavirus pneumonia.

**15.** An active pharmaceutical ingredient of a compound of Formula (I), wherein the active pharmaceutical ingredient comprises the amorphous form of the compound of Formula (I) according to any one of claims 1-7.

**16.** A pharmaceutical composition, wherein the pharmaceutical composition is composed of the active pharmaceutical ingredient according to claim 15 and a pharmaceutically acceptable excipient, and the pharmaceutically acceptable excipient comprises at least one of filler, binder, disintegrant, and lubricants.

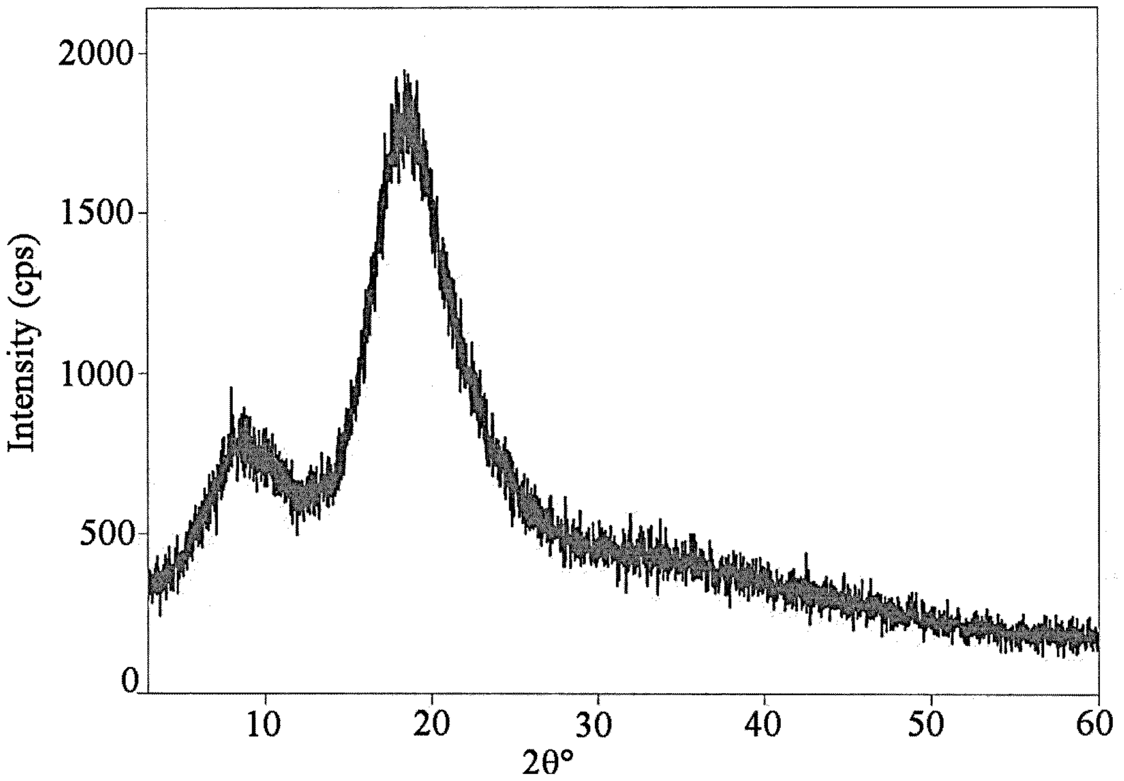


Figure 1

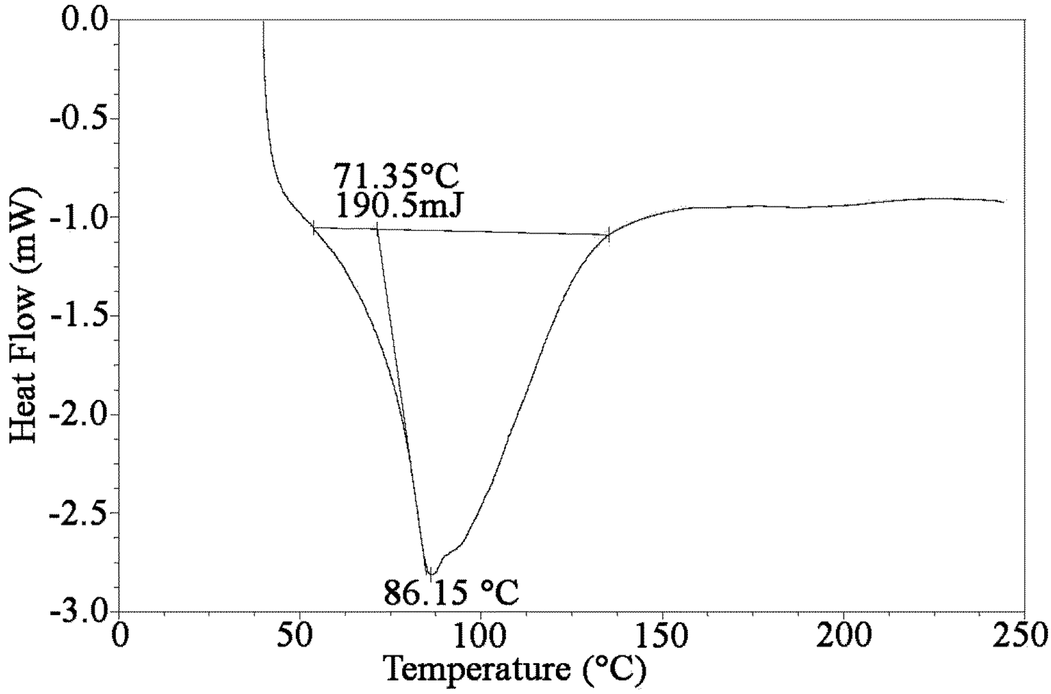


Figure 2

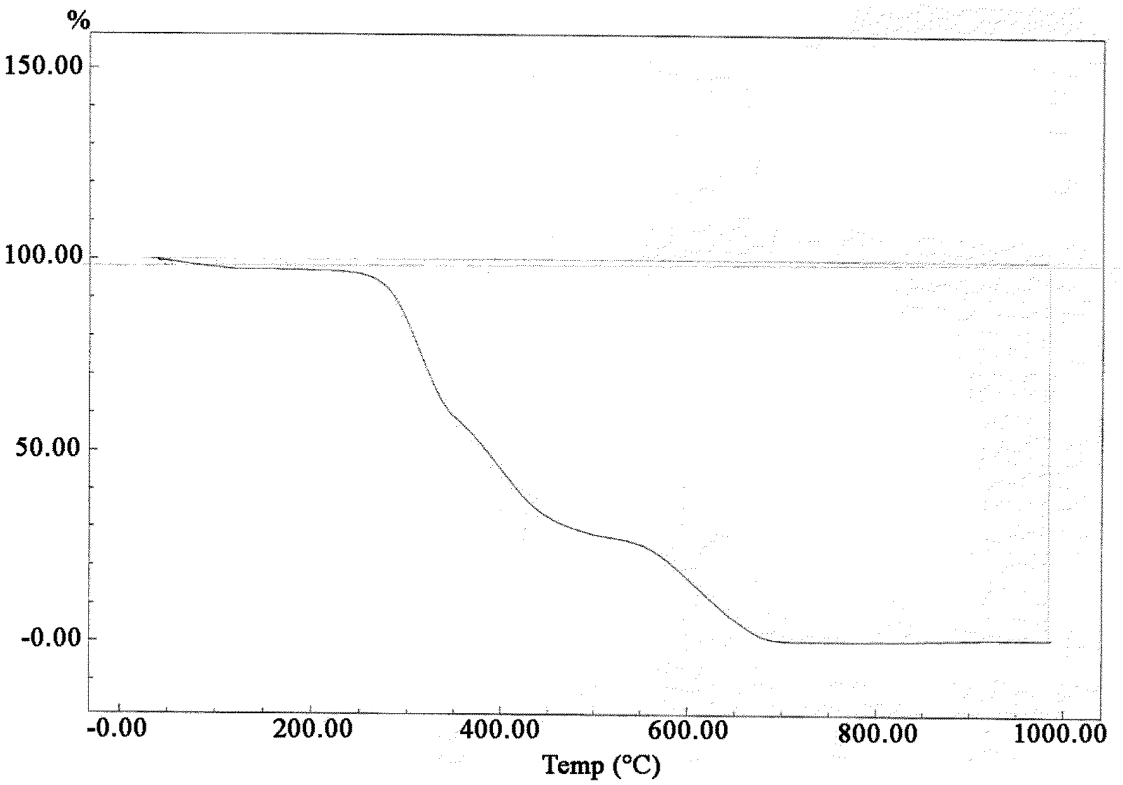


Figure 3

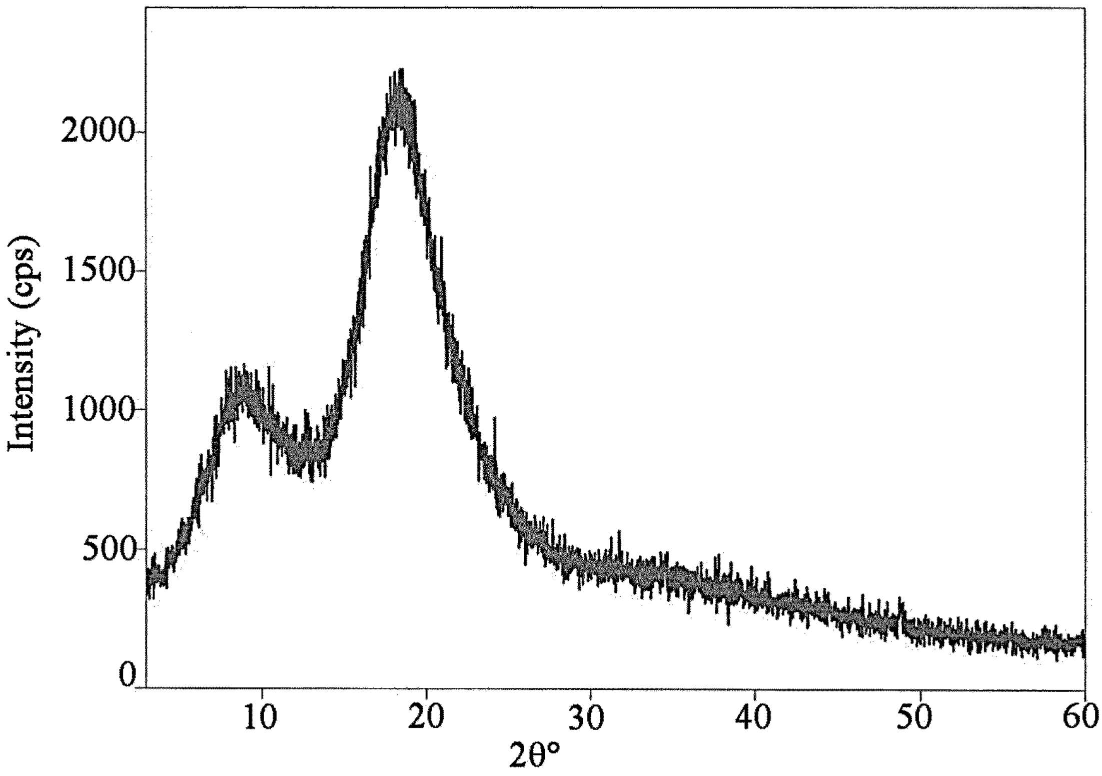


Figure 4

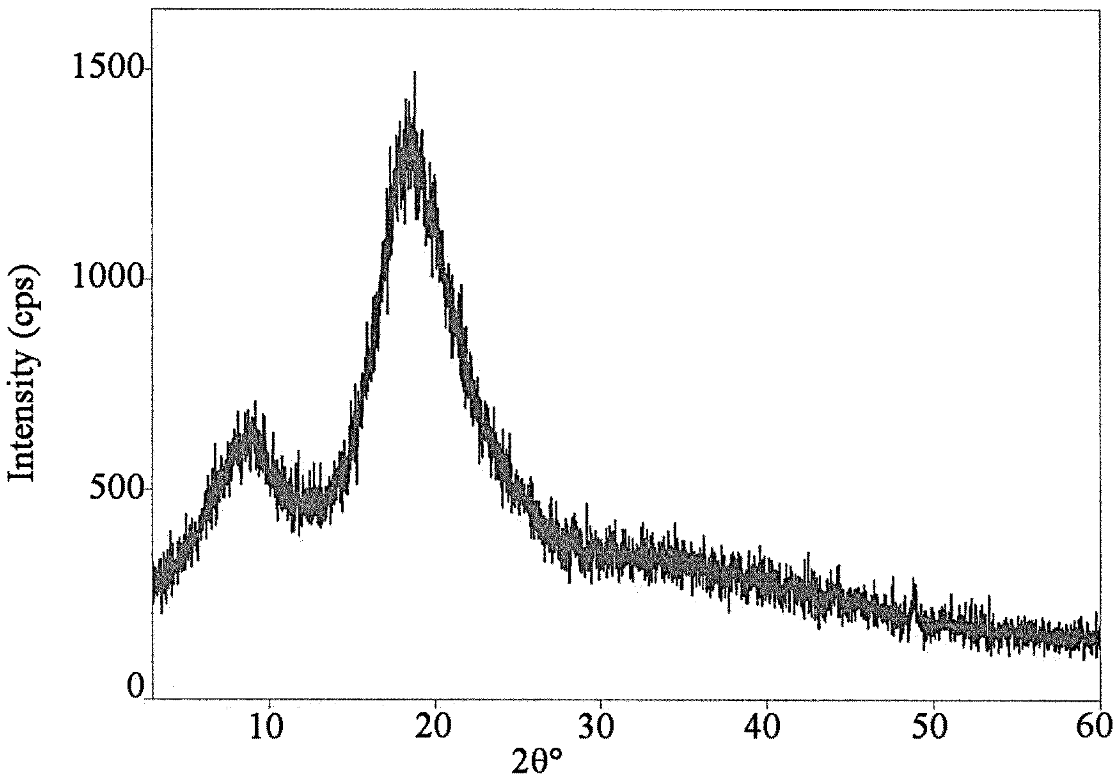


Figure 5

**INTERNATIONAL SEARCH REPORT**

International application No.
**PCT/CN2023/120437**

**A. CLASSIFICATION OF SUBJECT MATTER**

C07K5/087(2006.01)i； A61K38/06(2006.01)i； A61P31/14(2006.01)i

According to International Patent Classification (IPC) or to both national classification and IPC

**B. FIELDS SEARCHED**

Minimum documentation searched (classification system followed by classification symbols)

IPC： C07K A61K A61P

Documentation searched other than minimum documentation to the extent that such documents are included in the fields searched

Electronic data base consulted during the international search (name of data base and, where practicable, search terms used)

CNABS; CNTXT; ENTXT; DWPI; STNext Registry; STNext Caplus; CNKI: 广东众生睿创生物科技; 陈小新; 结构检索, Structure search, 无定型; amorphous; 3C-like protease; 3CL; 2923321-59-7; 2923310-64-7

**C. DOCUMENTS CONSIDERED TO BE RELEVANT**

| Category* | Citation of document, with indication, where appropriate, of the relevant passages | Relevant to claim No. |
|---|---|---|
| PX | CN 115594734 A (GUANGDONG RAYNOVENT BIOTECH CO., LTD.) 13 January 2023 (2023-01-13)<br>claims 12 and 14-15 | 1-16 |
| A | WO 2021226546 A1 (THE BOARD OF TRUSTEES OF THE LELAND STANFORD JUNIOR UNIVERSITY) 11 November 2021 (2021-11-11)<br>claims 25-59 and 65-71, and abstract | 1-16 |
| A | WO 2018042343 A2 (GLAXOSMITHKLINE INTELLECTUAL PROPERTY (NO.2) LIMITED) 08 March 2018 (2018-03-08)<br>claims 1-15, and description, Table 2 | 1-16 |
| A | WO 2021191827 A1 (GLAXOSMITHKLINE INTELLECTUAL PROPERTY DEVELOPMENGT LIMITED) 30 September 2021 (2021-09-30)<br>claims 1-31, and description, page 17, lines 10-15 | 1-16 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 December 2023** | **20 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**
**Information on patent family members**

International application No.
**PCT/CN2023/120437**

| Patent document cited in search report | Publication date (day/month/year) | Patent family member(s) | Publication date (day/month/year) |
|---|---|---|---|
| CN 115594734 A | 13 January 2023 | TW 202311246 A | 16 March 2023 |
| | | WO 2023036093 A1 | 16 March 2023 |
| WO 2021226546 A1 | 11 November 2021 | US 2023192660 A1 | 22 June 2023 |
| | | EP 4146267 A1 | 15 March 2023 |
| | | CA 3180177 A1 | 11 November 2021 |
| WO 2018042343 A2 | 08 March 2018 | TW 201817714 A | 16 May 2018 |
| | | WO 2018042343 A3 | 19 April 2018 |
| | | UY 37381 A | 23 March 2018 |
| WO 2021191827 A1 | 30 September 2021 | TW 202202167 A | 16 January 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022117124 W